# EUROPEAN PATENT APPLICATION

(11) **EP 4 166 646 A1**
(43) Date of publication of application: **19.04.2023**
(21) Application number: 20940148.8
(22) Date of filing: 11.06.2020
(51) Int. Cl.: C12N 1/04, C12N 1/20, C12R 1/225, C12R 1/01, A23L 33/135, A61K 35/744

(54) **METHOD FOR ISOLATING LACTIC ACID BACTERIA (LAB) FROM COMPLEX SAMPLES**

(71) Applicant: Universidad De La Frontera, Temuco 4811230 (CL)
(72) Inventor: DURÁN CUEVAS, Paola Andrea, Santa Olga Temuco (CL); CHÁVEZ JEREZ, Mariannys, Temuco (CL)
(74) Representative: Clarke Modet & Co.
(86) International application number: PCT/CL2020/050062
(87) International publication number: WO 2021/248255

(57) **Abstract**

Lactic acid bacteria (LAB) growth and isolation method in complex samples, comprising four steps: a) Fermentation; b) Inoculation of an MRS agar medium supplemented with Calcium Carbonate (CaCO3) and sorbic acid, with an aliquot obtained in a); c) Cultivation of the inoculated plates from step b) under microaerophilic conditions; and d) Selection of colonies by visual inspection, and quadrant streaking on MRS agar under microaerophilic conditions.

## Description

### FIELD OF THE INVENTION

The present invention belongs to the field of Microbiology, Biotechnology and Food. The present invention refers to a microbiological method that allows the growth, isolation and identification of lactic acid bacteria (LABs) from samples where the LABs are found in a low concentration, wherein said method comprises the following steps: (1) Fermentation, (2) inoculation on MRS agar, (3) Incubation in a microaerophilic atmosphere, and (4) Selection of colonies and quadrant streaking.

### BACKGROUND OF THE INVENTION

Lactic acid bacteria (LABs) are a large and heterogeneous group of Gram-positive bacteria, characterized by their strict fermentative metabolism, with lactic acid being the main byproduct during sugar fermentation. This bacterial group is of great importance for the biotechnology industry since they are part of starter cultures and are part of probiotics, widely used in the food industry. This particular group of bacteria comprises more than a dozen different genera, some of the best known being the genus *Lactococcus* and *Lactobacillus,* since they have been used as components of different products of the food industry. Due to the importance and versatility of this bacterial group, the number of LABs that are isolated from different environments continues to increase, and special culture media continue to be developed for the isolation of said bacteria from different types of samples.

The state of the art teaches that different culture media have been developed for the growth of this bacterial group. The standard culture medium for LAB isolation is MRS medium (Schillinger and Holzapfel. 2003. Culture media for lactic acid bacteria. Handbook of Culture Media for Food Microbiology, J.E.L. Corry et al. (Eds.), pp. 127-140). However, it has been shown that this type of bacteria grows well in this culture medium when they come from samples where the LABs are present in a large quantity in relation to other contaminating organisms, **but good yields have not been obtained when this bacterial group is present in low concentrations in complex samples.**

There are some efforts that have been made to improve the formulation of the MRS culture medium and the growth conditions of the samples have been optimized with the aim of optimizing the yields of LAB obtained. **However, there is an unmet need in the art for a robust method for isolating LABs from samples where these bacteria are at a low concentration.**

Specifically in relation to the isolation of bacteria from different samples, variations of growth conditions using MRS media have been described in patent documents and scientific publications (JP2019122296A; CN109337838A; KR20180072225A and US2017360052A1; Ruiz Rodriguez., et al. 2019. Diversity and Functional Properties of Lactic Acid Bacteria Isolated From Wild Fruits and Flowers Present in Northern Argentina; Saguibo., et al. 2019. Identification and characterization of lactic acid bacteria isolated from some medicinal and/or edible Philippine plants).

JP2019122296A describes a method for isolating lactic acid bacteria from the root of a plant called kudzu (*Pueraria Montana* var. lobata) and methods for the production of yogurt using said lactic acid bacteria as starter cultures are also disclosed. The described method includes an enrichment step using a culture medium for Lactic Acid Bacteria, however only reference is made to the fact that this culture medium is standard, and its components are not disclosed. Samples are grown in the culture medium containing cycloheximide and then grown at 25°C to 37°C for 24 to 48 hours. The enriched culture is then plated on a BCP agar plate.

CN109337838A discloses a method describing a preparation of aquatic products and methods of preparation thereof. The preparation method refers to a fermentation from a water sample obtained from a culture medium where aquatic animals live. The culture medium used was MRS (1% peptone; 0.5% meat extract, 0.4% yeast extract, 2% glucose, 0.1% Tween 80, 0.2% dipotassium phosphate, 0.2% sodium acetate. 0.5%, triammonium citrate 0.2%, magnesium sulfate 0.02%, manganese sulfate 0.005%, pH 6.2. The samples were incubated in said culture medium at 37°C for between 24 and 48h.

KR20180072225A discloses a method for the isolation of Lactic Acid Bacteria from kefir, which comprises inoculating the samples of this food in MRS culture medium and subsequently growing said cultures under strict anaerobic conditions at 37°C for 48 hours. After growth, LacticAcid Bacteria are selected in a first approximation by their colony shape and then selected by the gram test and catalase test. It is indicated that the selected colonies were passed through different passages in selective plates until obtaining pure cultures.

US2017360052A1 describes a protocol for the isolation of lactic acid bacteria from a food called Nuruk and related samples. The protocol includes a first rupture of the sample to later grow them in MRS medium supplemented with 0.01% cycloheximide and SBD (2% maltose, 0.3% yeast extract, 1.5% fresh yeast extract, 0.03% Tween 80, 0.6% casein peptone, pH 5.6. Growth conditions are 37°C for 48 hours.

Ruiz Rodriguez., et al., describes a protocol for the isolation of LacticAcid Bacteria from wild fruits and from flowers. The isolation and selection protocol for this type of bacteria includes: (1) Suspension of samples in 0.1% peptone water, (2) Serial dilutions and growth on MRS agar supplemented with 2% fructose or 2% glucose, (3) Incubation at 37°C for between 24 and 72 hours in anaerobic conditions, (4) Detection of lactic acid bacteria using calcium carbonate, and (5) Selection of LAB by morphological characteristics and catalase activity.

Saguibo., et al, describe a protocol for the isolation of lactic acid bacteria from medicinal and edible plants of the Philippines. The isolation and selection protocol for this type of bacteria includes: (1) Suspension of samples in 0.1% peptone water, (2) Serial dilutions and growth on MRS agar supplemented with 2% fructose or 2% glucose, (3) Incubation at 30°C for between 24 and 72 hours in anaerobic conditions, (4) Detection of lactic acid bacteria using calcium carbonate, and (5) Selection of LAB by morphological characteristics and catalase activity.

Although some of the prior art documents separately describe some of the steps and conditions of the method developed by the inventors, no method is taught that comprises each of the steps and technical elements described herein.

Specifically, no document described in the state of the art describes a method that makes it possible to obtain LAB isolates, when these are found in a concentration of less than 4% of microorganisms in a given sample, nor does any of said documents teach that microaerophilicity and the use of sorbic acid are conditions critical to be able to successfully isolate this type of bacteria in complex samples.

Therefore, the lack of efficient methods for the isolation of LABs from complex samples remains a technical problem, these being defined as those samples in which lactic acid bacteria are in a concentration of less than 4% in relation to the total.

### SUMMARY OF THE INVENTION

The present invention corresponds to a microbiological method that allows the growth, isolation and identification of lactic acid bacteria from samples where the LABs are found in a low concentration, where said method comprises the following steps: (1) Fermentation, (2) inoculation on MRS agar, (3) Incubation in a microaerophilic atmosphere, and (4) Selection of colonies and quadrant streaking.

### BRIEF DESCRIPTION OF THE FIGURES

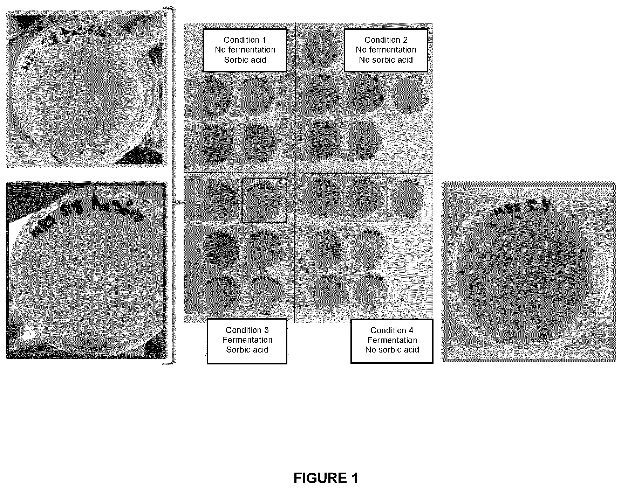

### Figure 1. Evaluation of the conditions, previous Fermentation and Presence of Sorbic Acid in the culture medium, on the growth and isolation of Lactic Acid Bacteria.

Four groups of cultures in Petri dishes with extended MRS agar from complex samples are visualized. Here two variables are combined, Previous Fermentation and Presence of Sorbic Acid in the medium, which generated four different conditions (1- No Fermentation + Sorbic Acid; 2- No Fermentation + No Sorbic Acid; 3- Fermentation + Sorbic Acid, 4- Fermentation + No Sorbic Acid), being the best 3, Previous Fermentation + Sorbic Acid. This last combination exhibited more colony growth of putative LABs than the others and at the same time showed much less growth of undesirable or opportunistic microorganisms. The central image exhibits the four different conditions: **1- upper left quadrant,** inoculation from non-fermented sample in medium enriched with sorbic acid; **2- upper right quadrant,** cultures from non-fermented sample and medium not enriched with sorbic acid; **3- lower left quadrant,** plates inoculated from fermented samples and sorbic acid-enriched medium; **4-lower right quadrant,** cultures inoculated from fermented sample, in a medium not enriched with sorbic acid. **Top left box with yellow edge:** enlargement of a plate from condition 3, where innumerable small-diameter colonies and dark spots on the agar are visible, a product of the dissolution of CaCOs from the medium, indicating the presence of LABs. **Bottom left box with red edge:** enlargement of the plate of condition 3 where around a dozen small, round colonies are visualized, with a dilution halo around them, revealing the presence of LABs. **Right box with orange edge:** enlargement of a plate completely covered by large, irregular colonies with a globular appearance, it is not possible to visualize the presence of LABs.

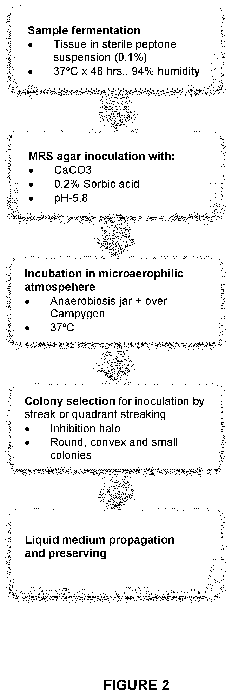

### Figure 2. Summary of the protocol developed in the present invention for the isolation of LABs from complex samples.

A schedule of steps of the Lactic Acid Bacteria Isolation Protocol from complex samples is shown.

### DETAILED DESCRIPTION OF THE INVENTION

In a first aspect, the present invention corresponds to a new method that allows the isolation of LABs from complex samples, which comprises the following steps:
1. **Fermentation:** The sample is incubated in a 0.1% sterile peptone solution, at 25-37°C for a maximum of 24-48 hours.
2. **Inoculation on MRS agar:** Serial dilutions are made from step 1, and these are plated on MRS agar enriched with 0.2% sorbic acid and 1% CaCO3, and the medium is adjusted to pH 5.8.
3. **Incubation in a microaerophilic atmosphere:** The plates are incubated in an atmosphere with a concentration of O₂ less than 15%, at 37°C for 48 hours.
4. **Selection of colonies and quadrant streaking:** The rounded, convex colonies and those that present a dilution halo around them are selected, and quadrant streaked until pure cultures are obtained.

In a preferred embodiment, the first step of the method corresponds to the inoculation of the sample from which the LABs are to be isolated in a 0.1% w/v sterile peptone solution.

In a more specific preferred embodiment, the sample is selected from the group consisting of: soil sample, river sediment sample, marine sediment sample, fresh water sample, salt water sample, and plant sample.

In an even more specific embodiment, the plant sample can correspond to parts of the plants such as: stem, leaves and flowers.

In another preferred embodiment, if the plant sample is from the endosphere (internal part of the plant), the outer part of the sample to be inoculated must be disinfected (preferably with ethanol), then the sample must be cut under sterile conditions and finally the sample is inoculated into the sterile peptone solution.

In a preferred embodiment, the samples from the fermentation step are grown for a period of time between 24 hours and a maximum of 48 hours.

In an even more specific preferred embodiment, the samples in the fermentation step are grown for between 30 and about 48 hours.

In another specific embodiment, the samples in the fermentation step are grown at a temperature between 25°C and 40°C.

In an even more specific embodiment, the samples in the fermentation step are grown at 37°C.

In a second embodiment, an aliquot from the fermentation step is inoculated on the MRS agar.

In another preferred embodiment, the MRS culture medium has the following composition: Peptone 1%; meat extract 0.5%, yeast extract 0.4%, glucose 2%, Tween 80 0.1%, dipotassium phosphate 0.2%, sodium acetate 0.5%, triammonium citrate 0.2%, magnesium sulfate 0.02%, manganese sulfate 0.005%.

In a more specific preferred embodiment, the MRS medium is supplemented with sorbic acid in a concentration of between 0.1% and 0.5%.

In a more specific embodiment, the sorbic acid concentration is 0.2%.

In a further embodiment the MRS medium is supplemented with a concentration of calcium carbonate (CaCO₃) from 0.1% to 2%.

In another more specific embodiment, the concentration of CaCOs is about 1%.

In another embodiment, the MRS medium is supplemented with 0.2% sorbic acid, and with CaCO₃ at 1%.

In a third further embodiment the MRS agar inoculated with the fermented sample (derived from steps 1 and 2) is grown in a microaerophilic atmosphere.

In a more specific embodiment, the O₂ concentration within the microaerophilic atmosphere is between 0.2% to 20.9%.

In a more specific preferred embodiment, the oxygen concentration within the microaerophilic atmosphere is between 8% to 20%.

In an even more specific preferred embodiment, the oxygen concentration is about 15%, preferably 15%.

In a preferred embodiment, the microaerophilic incubation is for a period of time between 48 hours and 72 hours.

In an even more specific preferred embodiment, the microaerophilic incubation is for a period between about 48 and about 72 hours.

In another specific embodiment, the microaerophilic incubation is at a temperature between 25°C and 40°C.

In an even more specific embodiment, the microaerophilic incubation is grown at 37°C.

In a fourth embodiment, after the microaerophilic incubation period, the colonies are macromorphologically observed and those that present a dilution halo around them, with a rounded, convex colony, are selected for subsequent characterizations. Selected colonies are quadrant streaked, inoculating said colonies on MRS agar and then said inoculated plates are grown under microaerophilic conditions as mentioned above.

In a last additional step, the pure colonies of BALs obtained are grown in liquid MRS culture medium, and then stored in a 25% glycerol solution at low temperatures (preferably -20 and/or -80).

In a further preferred embodiment, a cryopreservative medium is used for the storage of the isolated lactic acid bacteria, which may comprise glycerol or sucrose.

In a further preferred embodiment, the glycerol or sucrose may be in a concentration of between 10% and 50%, preferably 30%.

In the context of the present invention, standard microbiological methods and handling of microorganisms are understood as part of the knowledge of an expert, in order to be able to reproduce the invention. Representative methods are shown in the Examples attached later in this document that are intended to illustrate the present invention, but not to limit its scope.

### APPLICATION EXAMPLES

### Example 1: Evaluation of sorbic acid for the growth and isolation of lactic acid bacteria from complex samples

As a first approach to develop a robust method for the isolation of lactic acid bacteria from samples with a low concentration of LABs (less than 4%), the addition of sorbic acid to MRS agar was evaluated. Sorbic acid is used as an inhibitor of the LAB-competing microbiota, preventing its proliferation (Reuter *et al.*, 1985). In this way, the opportunistic bacteria that are in greater numbers and have a more accelerated growth rate, do not exhaust the nutrients in the medium, nor the space on the plate; leaving these nutrients available for the LABs. The evaluated conditions are shown in the following table:

**Table 1. Conditions resulting from the evaluation of the fermentation and the addition of sorbic acid on the growth of LABs.**

| **Condition** | **Culture medium** | **Previous Fermentation (Yes/No)** | **Sorbic Acid Addition (Yes/No)** |
|---|---|---|---|
| 1 | Agar MRS | No | Yes |
| 2 | Agar MRS | No | No |
| 3 | Agar MRS | Yes | Yes |
| 4 | Agar MRS | Yes | No |

When the culture medium is enriched with sorbic acid, important differences in microbial growth are manifested in the cultured plates to obtain BALs. This is because this compound prevents the proliferation of unwanted microorganisms. This can be seen in Figure 1, where a set of plates with MRS enriched or not, with sorbic acid is displayed and where some of these were inoculated from previously fermented samples, giving rise to 4 different conditions.

In the quadrants that reflect conditions 1 and 2, microbial growths from fresh samples are observed. In both cases few colonies are observed, however, for condition 2 where the medium was not enriched with sorbic acid, the presence of opportunistic microorganisms can be seen. Similar to this we have conditions 3 and 4, where the difference with respect to the previous conditions lies in the fact that the samples were fermented before inoculation. So, we have a very similar result; but increased as a result of the previous fermentation that the samples underwent. In quadrant 4, plates totally covered by opportunistic bacteria are visualized, in contrast to quadrant 3, which was enriched with sorbic acid, exhibits plates with many colonies with putative LAB morphology and very little or no growth of opportunistic microorganisms (almost none).

LABs are slow-growing and have high nutritional requirements, therefore, if it is necessary to isolate them from complex samples where microorganisms with a higher growth rate abound, it is necessary to add an antimicrobial that limits their growth. This way they do not deplete the nutrients and space in the medium, before the LABs can thrive. On the other hand, the previous fermentation contributes to the probabilities of success of the LAB isolation, since the number of microorganisms increases with respect to the fresh sample. It must be taken into account that in complex samples these are found in a very low proportion with respect to the general microbiota.

In figure 1 the photos that are enlarged and labeled with R, are cultures from the Rhizosphere, which is nothing more than the soil attached to the root of the plant; but it does not contemplate the root, only the soil that surrounds it. So as an example, for two plates planted with the exact same conditions, coming from the Rhizosphere, at a dilution of 10⁻⁴, with the only difference that one had the medium enriched with sorbic acid and the other did not, 10 CFU of putative LABs were observed for the first case, however, for the culture in which sorbic acid was not added, the plate was completely covered (Countless CFUs) of competing bacteria. On the other hand, the plate seeded from an aliquot of 50 µL of the dilution of 10⁻² in the medium with sorbic acid, approximately 288 CFU were counted, putative LABs (Figure 1).

To further visualize the superior technical effect of the specific conditions used in the current protocol, a summary table is shown below (Table 2) where the negative results of the protocols that were used previously (P1-P4) to the protocol used successfully in the present methodology (P6) are evident. These protocols were generated from different modifications that started in the Traditional protocol and finally gave rise to the successful protocol (P6). Identifications were made by comparing the 16S rRNA gene sequences with the Genbank sequences by BLASTN and also by proteomic analysis using MALDI-Toff.

Besides the foregoing, in the following Table 3, a summary of each of the conditions tested for each of said protocols (P1-P6) is shown.

**Table 2. Identifications of isolates resulting from different modified protocols for the isolation of lactic acid bacteria, based on partial sequencing of the 16S rRNA gene and comparison with Genbank sequences by BLASTN and proteomic analysis by MALDI-Toff.**

| **Protocol** | **Identification pathway** | **Strain Code** | **Source** | **ID** | **similarity** |
|---|---|---|---|---|---|
| | | E1I | Endosphere hazelnut | *Bacillus subtilis* | 96% |
| | | E1II | Endosphere hazelnut | *Lysinibacillus boronitolerans* | 93% |
| | | E1III | Endosphere hazelnut | *Bacillus subtilis* | 99% |
| Traditional | | E2I | Endosphere hazelnut | *Bacillus megaterium* | 95% |
| | | E2 II | Endosphere hazelnut | *Bacillus paranycoides* | 99% |
| | sequence similarity | e3i | Endosphere hazelnut | *Bacillus australimaris* | 99% |
| | | E3II | Endosphere hazelnut | *Bacillus aerius* | 99% |
| | | E3III | Endosphere hazelnut | *Bacillus estratosphericus* | 99% |
| | | P47PI | Phyllosphere hazelnut | *Bacillus tropicus* | 99% |
| | | P47AM | Phyllosphere hazelnut | *Bacillus subtilis* | 97% |
| | | P47GI | Phyllosphere hazelnut | *Bacillus megaterium* | 99% |
| | | R1 | Rhizosphere hazelnut | *Bacillus thuringiensis* | 98% |
| | | R2 | Rhizosphere hazelnut | *Bacillus wiedmannii* | 99% |
| | | R3 | Rhizosphere hazelnut | *Bacillus thuringiensis* | 99% |
| P1 | Maldi-Toff (Score on a scale of 0,000-3,000; it is considered reliable from 2,000) | 1 | Endosphere hazelnut | *Bacillus cereus* | 2.41 |
| | | 2 | Phyllosphere hazelnut | *Bacillus cereus* | 2.42 |
| | | 4 | Phyllosphere hazelnut | *Bacillus cereus* | 2.4 |
| | | 6 | Phyllosphere hazelnut | *Bacillus mycoides* | 2.13 |
| | | 7 | Phyllosphere hazelnut | *Bacillus cereus* | 2.42 |
| | | 9 | Endosphere hazelnut | *Bacillus cereus* | 2.18 |
| | | 10 | Endosphere hazelnut | *Bacillus cereus* | 2.06 |
| | | 15 | Phyllosphere hazelnut | *Bacillus cereus* | 2.14 |
| | | 23 | Rhizosphere hazelnut | *Bacillus mojavensis* | 2.1 |
| | | 24 | Rhizosphere hazelnut | *Bacillus subtilis ssp subtilis* | 2.05 |
| | | FS 3A | Phyllosphere hazelnut | *Staphylococcus capitis ssp capitis* | 2.1 |
| P2 | | 10 | Phyllosphere hazelnut | *Lysinibacillus* | 96% |
| | sequence similarity | 12 | Phyllosphere hazelnut | *Bacillus* | 93% |
| | | 7a | Rizhosphere | *Bacillus* | 99% |
| | | 17 | Rizhosphere | *Lysinibacillus* | 98% |
| | | 18 | Rizhosphere | *Bacillus* | 96% |
| P3 | pH screening | | Endosphere, Phyllosphere, Rhizosphere | It was not possible to isolate putative BALs. | |
| P4 | | BT₀R₃F | Phyllosphere | *Staphylococcus auricularis* | 99% |
| | | I-A | Rhizosphere | *Staphylococcus saprophyticus* | 99% |
| | | VM | Rhizosphere | *Staphylococcus epidermidis* | 99% |
| | | MM | Rhizosphere | *Staphylococcus sp.* | 99% |
| | | CoP | Rhizosphere | *Staphylococcus sp.* | 99% |
| | sequence similarity | IAa | Rhizosphere | *Staphylococcus sp.* | 99% |
| | | IIAc1 | Rhizosphere | *Staphylococcus sp.* | 99% |
| | | IIAb | Rhizosphere | *Staphylococcus sp.* | 99% |
| | | BT₀R₁F-2 | Phyllosphere | *Bacillus sp.* | 98% |
| | | BT₀R₁F-1 | Phyllosphere | *Staphylococcus sp.* | 99% |
| | | TT₀R₃F-2 | Phyllosphere | *Staphylococcus sp.* | 99% |
| | | TT₀R₃F-3 | Phyllosphere | *Staphylococcus sp.* | 99% |
| | | TT₀R₃F-4 | Phyllosphere | *Staphylococcus sp.* | 99% |
| P6 | Maldi-Toff | A | Endosphere | *Lactobacillus paraplantarum* | 2.13 |
| | | B | Phyllosphere | *Bacillus subtilis* | 99% |
| | sequence similarity | C | Rhizosphere | *Bacterium endosymbiont of Onthofagus Taurus* | 99% |
| | | F | Endosphere | *Lactococcus garvieae* | 100% |
| | | H | Rhizosphere | *Lactococcus garvieae* | 99% |
| | | J | Rhizosphere | *Lactococcus garvieae* | 100% |
| | | K | Rhizosphere | *Enterococcus mundtii* | 99% |

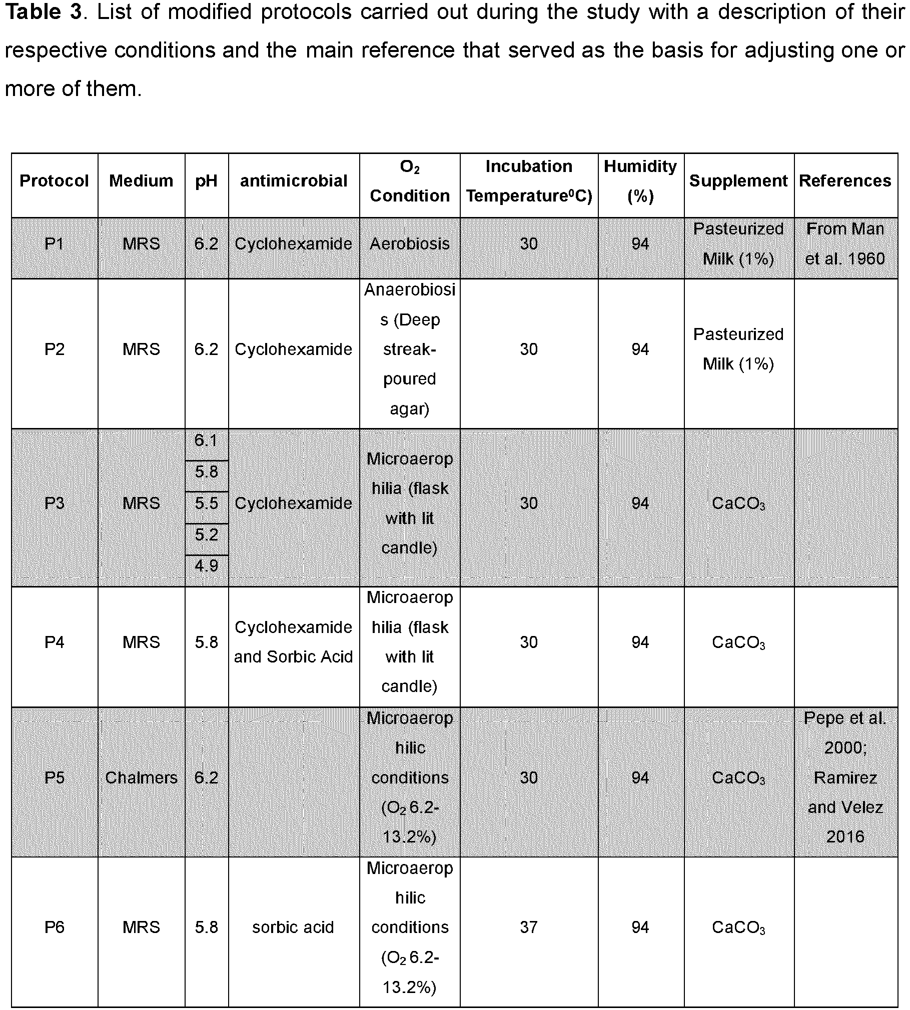

## Claims

1. Lactic acid bacteria (LAB) growth and isolation method in complex samples, wherein the method comprises four steps:
a) Fermentation;
b) Inoculation of an MRS agar medium supplemented with Calcium Carbonate (CaCO3) and sorbic acid, with an aliquot obtained in a);
c) Cultivating the inoculated plates from step b) under microaerophilic conditions; and
d) Selection of colonies by visual inspection, and quadrant streaking on MRS agar under microaerophilic conditions.

2. The production method according to claim 1, wherein the fermentation step consists of inoculating a sample in a 0.1% sterile peptone solution, at a temperature between 25°C-37°C for between 24 and 48 hours.

3. The production method of claims 1 or 2, wherein the strain in the fermentation step the temperature is 37°C and the incubation time is 48 hours.

4. The production method according to any of claims 1 to 3, wherein in step b) serial dilutions are made using the samples obtained from step a), and these are seeded on an MRS agar enriched with sorbic acid and CaCO3, and the medium is adjusted to pH 5.8.

5. The production method according to any of claims 1 to 4, wherein the concentration of sorbic acid is 0.2% and the concentration of CaCOs is 1%.

6. The production method according to any of claims 1 to 5, wherein in step c) the plates are incubated in an atmosphere with a concentration of O₂ less than 15%, at 37°C for 48 hours.

7. The production method according to any of claims 1 to 6, wherein in step d) the lactic acid bacteria colonies are selected according to their macromorphological characteristics.

8. The production method according to any of claims 1 to 7, wherein in step d) the analyzed characteristics of the colonies are the rounded and convex shape of the colony, and that they also present a halo of dilution around it.

9. The production method according to claim 8, wherein once the colonies in step d) are selected, they are purified by quadrant streaking on MRS agar with the same characteristics used previously.
